# EUROPÄISCHE PATENTSCHRIFT

(11) **EP 1 119 341 B1**
(45) Veröffentlichungstag und Bekanntmachung des Hinweises auf die Patenterteilung: **01.12.2004**
(21) Anmeldenummer: 99950539.9
(22) Anmeldetag: 22.09.1999
(51) Int. Cl.: A61K 7/16

(54) **FLÜSSIGES ZAHNREINIGUNGSGEL**
LIQUID TOOTH CLEANING GEL
GEL DENTIFRICE LIQUIDE

(30) Priorität: 01.10.1998 DE 19845247
(43) Veröffentlichungstag der Anmeldung: 01.08.2001
(73) Patentinhaber: Henkel Kommanditgesellschaft auf Aktien, 40589 Düsseldorf-Holthausen (DE)
(72) Erfinder: LEINEN, Hans-Theo, D-40229 Düsseldorf (DE); GREGORI, Dorothea, D-41470 Neuss (DE); CARBO, Maria, Rosa, E-08026 Barcelona (ES); PUJOL GALIANO, Miracle, E-08329 Teia (ES)
(86) Internationale Anmeldenummer: PCT/EP1999/007043
(87) Internationale Veröffentlichungsnummer: WO 2000/019970

(56) Entgegenhaltungen:
- WO-A-94/01080
- WO-A-95/22958
- DE-A- 2 250 078
- DE-A- 3 527 280
- DE-A- 4 222 739
- DE-A- 19 522 750
- FR-A- 2 183 228

## Beschreibung

Die Erfindung betrifft flüssige Zahnreinigungsmittel mit einem Gehalt an Poliermitteln, Feuchthaltemitteln und Wasser, die weitgehend transparent bis klar sind und ein günstiges rheologisches Verhalten für die Dosierung aus flexiblen Kunststofffläschchen aufweisen.

Es sind bereits flüssige Zahnreinigungsmittel bekannt, die sich aus flexiblen Kunststofffläschchen unter leichtem Druck ausbringen lassen, z.B. aus WO 94/01080 A1. Diese Produkte weisen ein weißes bis stark trübes Aussehen auf. Um solche Produkte transparent oder klar zu formulieren, ist es erforderlich, daß der flüssige Träger aus Wasser, Feuchthaltemitteln und ggf. gelösten Inhaltsstoffen einen dem Poliermittel ähnlichen Brechungsindex aufweist. Aus WO 95/22958 war z.B. eine transparente Formulierung bekannt, die einen Gehalt von mehr als 50 Gew.-% Sorbit aufweist. Diese flüssigen Zahngele zeigen jedoch eine noch nicht befriedigende Kältestabilität der Transparenz. Aus WO 94/01080 A1 war auch transparentes Flüssigzahnreinigungsmittel bekannt, das ca. 21 Gew.-% Sorbit, 30 Gew.-% Glycerin und 5 Gew.-% Ethanol enthält. Diese flüssigen Zahnreinigungsmittel sind sehr niedrigviskos und zeigen Viskositäten deutlich unter 10000 mPa·s (25° C).

Die Aufgabe der vorliegenden Erfindung bestand darin, ein noch flüssiges Zahnreinigungsgel vorzuschlagen, das zwar eine vorzügliche Kältestabilität der Transparenz aufweist, aber doch nicht zu flüssig ist und beim Dosieren auf eine Zahnbürste nicht zu rasch und zu tief in die Borsten einsinkt.
Diese Aufgabe wurde durch Verwendung eines spezifischen Feuchthaltemittelgemisches gelöst:

Gegenstand der Erfindung ist ein flüssiges, transparentes Zahnreinigungsgel mit einem Gehalt von
10 - 15 Gew.-% Kieselsäure-Polierkomponenten
55 - 70 Gew.-% Feuchthaltemittel (a + b + c)
15 - 30 Gew.-% Wasser und
2 - 10 Gew.-% weiterer Zahnpasteninhaltsstoffe,
dadurch gekennzeichnet, daß als Feuchthaltemittel ein Gemisch aus Sorbit (a), Glycerin (b) und Polyethylenglycol (c) im Gewichtsverhältnis (a) : (b) : (c) = 10 : (7 - 8) : (0,5 - 1,5) darin enthalten ist.

Als flüssig im Sinne der vorliegenden Erfindung wird eine Viskosität von ca. 10 000 - 100 000 mPa·s (20° C, gemessen mit einem Brookfield Rotationsviskosimeter RVF, Spindel 3/4 bei 4 RPM, entsprechend einer Scherrate von D = 4s⁻¹) verstanden, die ein leichtes Dosieren aus einem flexiblen Spendefläschchen ermöglicht und ein langsames Einsinken des Gels in die Borsten der Zahnbürste bewirkt.

Die Transparenz soll wenigstens so hoch sein, daß durch eine ca. 1 cm dicke Schicht des Zahngels (z.B. in einer Klarglas-Küvette von 1 x 1 x 4 cm Kantenlänge) eine Schrift mit ca. 4 mm Buchstabenhöhe und 3 mm Buchstabenbreite noch gut lesbar ist.

Als Kieselsäure-Polierkomponenten eignen sich alle als Putzkörper bekannten Gelkieselsäuren, Hydrogelkieselsäuren und Fällungskieselsäuren. Gelkieselsäuren werden durch Umsetzung von Natriumsilikatlösungen mit starken, wäßrigen Mincralsäuren unter Ausbildung eines Hydrosols, Alterung zum Hydrogel, Waschen und Trocknen hergestellt. Erfolgt die Trocknung unter schonenden Bedingungen auf Wassergehalt von 15 bis 35 Gew.-%, so werden die sogenannten Hydrogelkieselsäuren erhalten, wie sie z.B. aus US 4,153,680 bekannt sind. Durch Trocknung auf Wassergehalte unterhalb 15 Gew.-% erfolgt eine irreversible Schrumpfung der vorher lockeren Struktur des Hydrogels zur dichten Struktur des sog. Xerogels. Solche Xerogelkieselsäuren sind z.B. in US 3,538,230 beschrieben.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Herstellung von Fällungskieselsäuren sind z.B. in DE-OS 25 22 486 und in DE-OS 31 14 493 beschrieben. Bevorzugt geeignet ist eine gemäß DE-OS 31 14 493 hergestellte Fällungskieselsäure mit einer BET-Oberfläche von 15 - 110 m²/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10 - 20 Gew.-% des Zahngels. Bevorzugt geeignete Fällungskieselsäuren dieser Art weisen außerdem gerundete Ecken und Kanten auf und sind unter der Handelsbezeichnung Sident®12 DS (DEGUSSA) erhältlich.

Andere Fällungskieselsäuren dieser Art sind Sident 8 (DEGUSSA) und Sorbosil AC 39 (Crosfield Chemicals). Diese Kieselsäuren zeichnen sich durch eine geringere Verdickungswirkung und eine etwas höhere mittlere Teilchengröße von 8 - 14 µm bei einer spezifischen Oberfläche von 40 - 75 m²/g (nach BET) aus und eignen sich besonders gut für flüssige Zahngele gemäß der vorliegenden Erfindung.

Weitere Poliermittel. insbesondere solche. die einen Brechungsindex aufweisen, der von 1.45 um mehr als 0.1 abweicht, sind bevorzugt nicht oder allenfalls in Mengen von weniger als 2 Gew.-% enthalten.

Von entscheidender Bedeutung für die Rheologie und Transparenz der erfindungsgemäßen flüssigen Zahnreinigungsgele ist die Zusammensetzung der flüssigen Trägerphase aus Wasser und Feuchthaltemitteln. Die Gesamtmenge von 55 - 70 Gew.-% an Feuchthaltemitteln setzt sich aus Sorbit (a), Glycerin (b) und Polyethylenglycol (c) in einem relativ engen Mengenverhältnis von (a) : (b) : (c) = 10 : (7 - 8) : (0.5 - 1.5) zusammen. Bei einem bevorzugten Gehalt von 30 - 35 Gew.-% Sorbit ergibt sich daher ein Gehalt von 21 - 28 Gew.-% an Glycrin und von 1,5 - 5,25 Gew.-% an Polyethylenglycol.

Wasser (d) und Feuchthaltemittel (a + b + c) sind bevorzugt in einem Gewichtsverhältnis von (d) : (a + b + c) = 1 : (2 - 3) enthalten. Bei einem bevorzugten Gehalt von 55 - 65 Gew.-% an Feuchthaltemitteln liegt der Wassergehalt bei 20 - 25 Gew.-%. Der Wassergehalt ergibt sich dabei als Summe des durch Rohstoffe wie z.B. 70 %iges Sorbit oder 86 %iges Glycerin eingetragenen und des separat zugesetzten Wassers. Die angegebenen Mengen an Sorbit und Glycerin beziehen sich auf eine wasserfreie Aktivsubstanz.

Zusätzlich zu den genannten obligatorischen Komponenten können die erfindungsgemäßen Zahnreinigungsgele weitere Zahnpasteninhaltsstoffe enthalten, die in Art und Menge die Transparenz nicht negativ beeinflussen. Solche Inhaltsstoffe sind z.B. Bindemittel, Tenside, Geschmackstoffe, Wirkstoffe gegen Zahn- und Zahnfleischerkrankungen, z.B. Fluorverbindungen, Antizahnsteinwirkstoffe, antibakterielle Stoffe (z.B. Biguanide, Triclosan), Vitamine, Panthenol und andere Wirksubstanzen.

Schließlich eignen sich die erfindungsgemäßen transparenten Zahnreinigungsgele auch hervorragend dazu. durch lösliche Farbstoffe und/oder gefärbte Pigmente zu Produkten mit ansprechendem Aussehen zu gelangen. Als gefärbte Pigmentpartikel eignen sich besonders gefärbte Kieselsäurepartikel, wie sie z.B. unter der Verkaufsbezeichnung Sorbosil BFG 51, BFG 52 und BFG 53 oder Sorbosil SD 2352 im Handel sind. Es können auch Gemische unterschiedlich gefärbter Pigmentpartikel verwendet werden. Solche z.B. kräftig orange, rot oder blau gefärbten Gelkieselsäure-Partikel können in Mengen von 0.1 - 1.0 Gew.-% in den erfindungsgemäßen Zahnreinigungsgelen enthalten sein.

Als Bindemittel können z.B. natürliche und/oder synthetische wasserlösliche Polymere wie Alginate, Carragheenate, Traganth, Stärke und Stärkeether, Celluloseether wie z.B. Carboxymethylcellulose (Na-Salz), Hydroxyethylcellulose, Methylhydroxypropylcellulose, Guar, Akaziengum, Agar-Agar, Xanthan-Gum, Succinoglycan-Gum, Johannisbrotmehl, Pectine, wasserlösliche Carboxyvinylpolymere (z.B. Carbopol®-Typen), Polyvinylalkohol, Polyvinylpyrrolidon, Polyethylenglycole, insbesondere solche mit Molekulargewichten von 1 500 - 1 000 000 eingesetzt werden.

Besonders gut geeignete Bindemittel sind Xanthan-Gum, Carboxymethylcellulose, Polyvinylpyrrolidon und Gemische dieser wasserlöslichen Polymeren, die in einer Menge bis zu 0,5 Gew.-% enthalten sein können.

Geeignete Tenside sind z.B. lineare Natriumalkylsulfate mit 12 - 18 C-Atomen in der Alkylgruppe. Diese Stoffe weisen zusätzlich eine enzymhemmende Wirkung auf den bakteriellen Stoffwechsel des Zahnbelags auf. Weitere geeignete Tenside sind Alkalisalze, bevorzugt Natriumsalze von Alkylpolyglycolethersulfat mit 12 - 16 C-Atomen in der linearen Alkylgruppe und 2 - 6 Glycolethergruppen im Molekül, von linearem Alkan(C ₁₂-C₁₈)-sulfonat, von Sulfobernsteinsäuremonoalkyl(C₁₂-C₁₈)-estern, von sulfatierten Fett-säuremonoglyceriden, sulfatierten Fettsäurealkanolamiden, Sulfoessigsäurealkyl(C₁₂-C₁₆)-estern, Acylsarcosinen, Acyltauriden und Acylisethionaten mit jeweils 8 - 18 C-Atomen in der Acylgruppe. Auch zwitterionische, ampholytische und nichtionische Tenside sind geeignet. z.B. Oxethylate von Fettsäuremono- und -diglyceriden. von Fettsäure-Sorbitanestern und Alkyl(oligo)-Glucoside.

Bevorzugt geeignet sind Natrium-laurylsulfat und ein Gemisch aus Natrium-laurylsulfat und Cocoamidopropyl-betain.

Als Geschmackstoffe werden üblicherweise Aromen und Süßungsmittel verwendet. Geeignete Aromen sind z.B. Pfefferminzöl, Krauseminzöl, Eukalyptusöl, Anisol, Fenchelöl, Kümmelöl, Methylacetat, Zimtaldehyd, Anethol, Vanillin, Thymol und andere natürliche oder naturidentische ätherische Öle oder auch synthetische Geschmackstoffe verwendet.

Als Süßungsmittel eignen sind z.B. Saccharin-Natrium, Natrium-Cyclamat, Sucrose, Lactose, Maltose oder Fructose.

Weitere übliche Zahnpasteninhaltsstoffe sind z.B.
- Konservierungsmittel und antimikrobielle Stoffe, z.B. p-Hydroxybenzoesäuremethyl, -ethyl- oder propylester, Natriumsorbat, Natriumbenzoat, Bromchlorophen, Triclosan, Hexetidin, Phenyl-salicylsäureester, Biguanide z.B. Chlorhexidin und Peroxide
- Fluorverbindungen wie z.B. Na-Fluorid, Zink-fluorid, Na-Monofluorophosphat, Aminfluorid u.a.
- Puffersubstanzen, z.B. primäre, sekundäre oder tertiäre Alkaliphosphate, Citronensäure/Na-Citrat,
- wundheilende und entzündungshemmende Stoffe wie z.B. Harnstoff, Allantoin, Panthenol, Alkali-Rhodanide, Kamillewirkstoffe, (Azulene) und Acetylsalicylsäurederivate.
- Vitamine, z.B. Retinol, Tocopherol oder Ascrobinsäure und
- Mineralsalze wie z.B. Mangan-, Zink- oder Magnesiumsalze.

Alle diese fakultativen Zahnpasteninhaltsstoffe sind zusammen in einer Menge von etwa 2 - 10 Gew.-% in den erfindungsgemäßen Zahngelen enthalten.

Die folgenden Beispiele sollen den Erfindungsgegenstand näher beschreiben:

### Beispiele

Es wurden flüssige Zahnreinigungsgele der folgenden Zusammensetzung hergestellt:

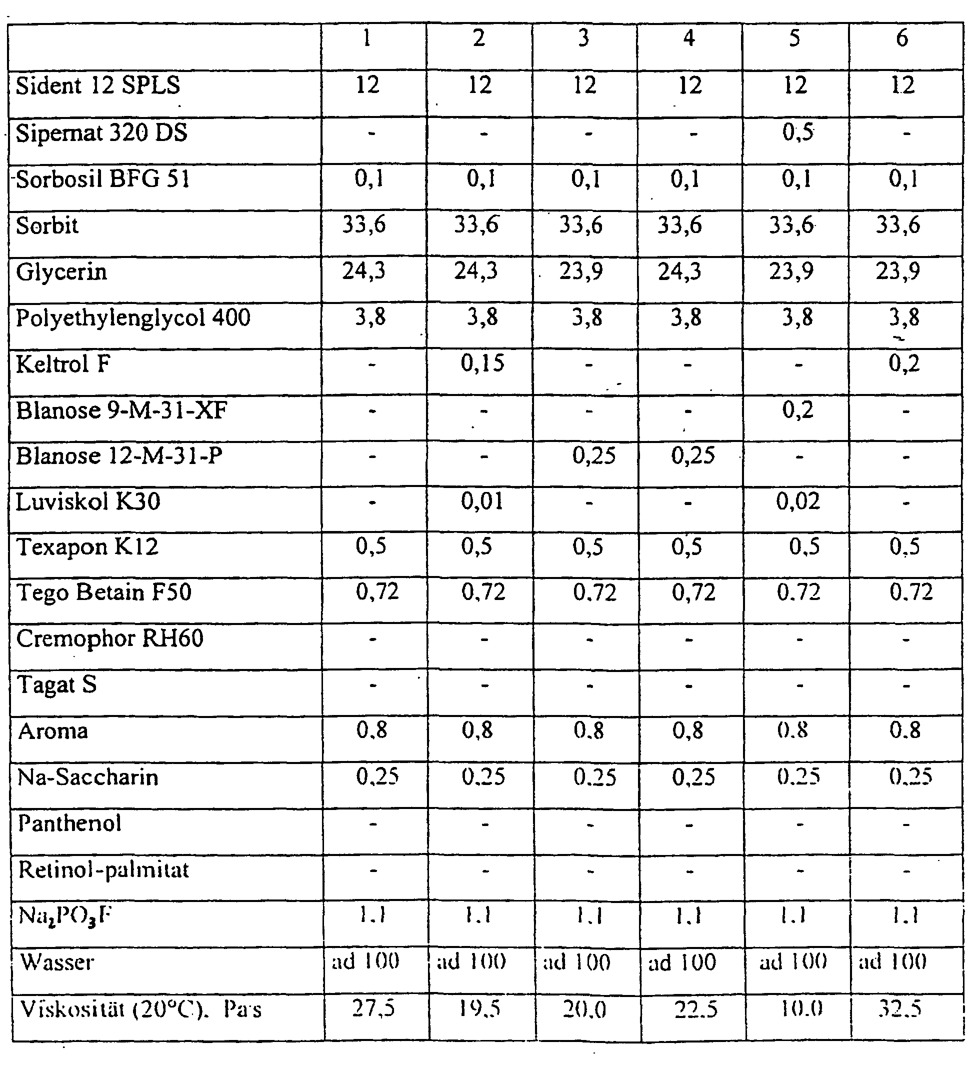

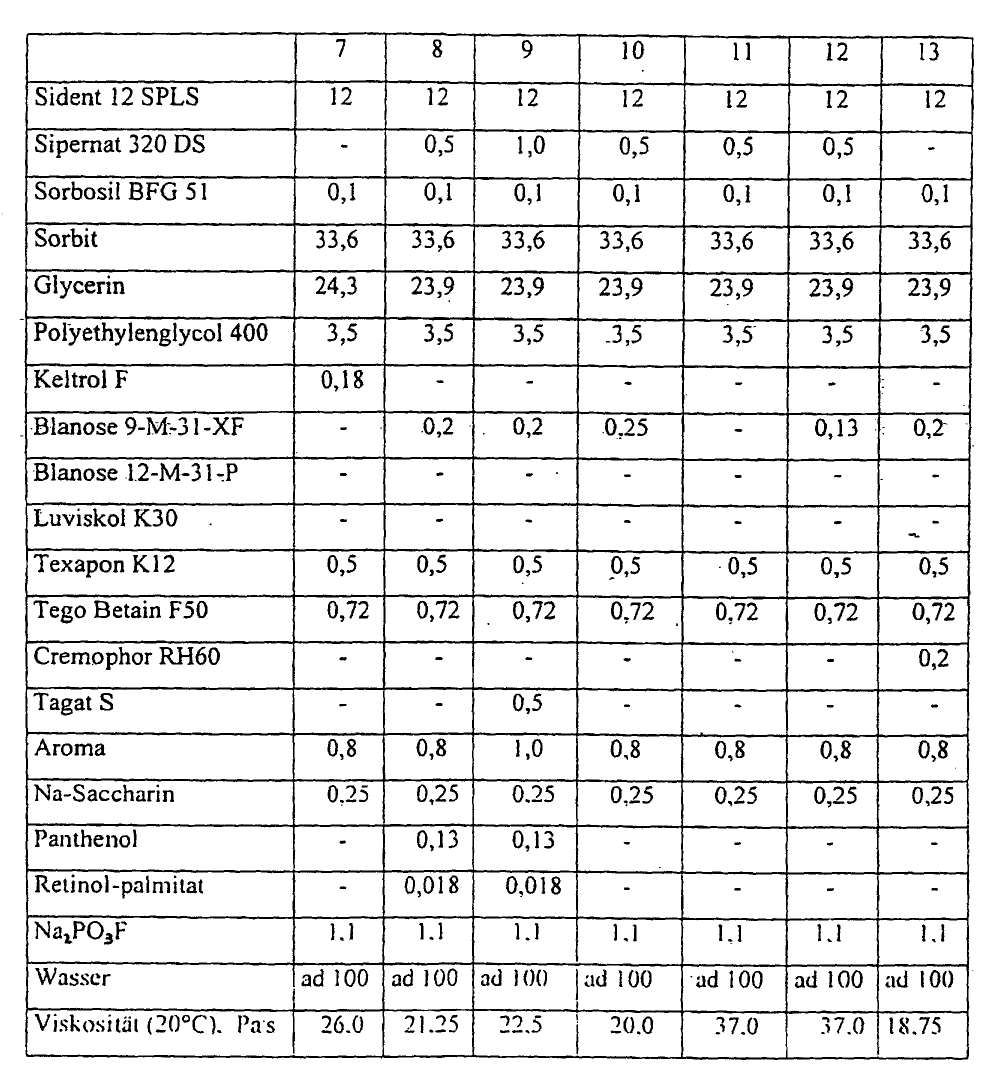

Alle Beispiele stellen transparente Gele dar, die auch nach 4 Wochen Lagerzeit bei 25°C ihre Transparenz behalten.

Es wurden folgende Handelsprodukte verwendet:
- Sident®12 SPLS :: Fällungskieselsäure
Spez. Oberfläche (BET) : 80 m²/g
Hersteller: DEGUSSA AG
- Sipernat®320 DS :: Fällungskieselsäure
Spez. Oberfläche (BET) : 170 m²/g
Hersteller: DEGUSSA AG
- Sorbosil® BFG 51 :: gefärbte, agglomerierte Fällungskieselsäure
mittlere Teilchengröße 310 µm
(min 90 % 106 - 710 µm)
- Keltrol® F :: Xanthan-Gum
Hersteller: Fa. KELCO. Brüssel
- Blanose® 9-M-31-XF :: Na-Carboxymethylcellulose
Subst.-Grad 0,8 - 0,95, Viskosität 100-300 mPa·s (1 %ig)
Hersteller: HERCULES
- Blanose® 12-M-31-XP :: Na-Carboxymethylcellulose
Subst.-Grad 1,2, Viskosität 100-300 mPa·s (1 %ig)
Hersteller: HERCULES
- Luviskol® K30 :: Polyvinylpyrrolidon, MG : ca. 40 000
Hersteller: BASF AG
- Texapon® K12 :: Na-Laurylsulfat
(HENKEL KGaA)
- Tego Betain® F50 :: N,N-Dimethyl-N-kokosamidopropylammoniumacetobetain (ca. 36 % AS)
Hersteller: TEGO-Cosmetics
- Cremophor® RH60 :: PEG 60 Hydrogenated Castor Oil
(BASF AG)
- Tagat® S :: Polyoxyethylen-(20)-glycerinmonostearat
Hersteller: TEGO-Cosmetics

## Patentansprüche

1. Flüssiges, transparentes Zahnreinigungsgel mit einem Gehalt von
10 - 15 Gew.-% Kieselsäure-Polierkomponenten
55 - 70 Gew.-% Feuchthaltemittel
15 - 30 Gew.-% Wasser und
2 - 10 Gew.-% weiterer Zahnpasteninhaltsstoffe,
**dadurch gekennzeichnet, daß** als Feuchthaltemittel ein Gemisch aus Sorbit (a), Glycerin
(b) und Polyethylenglycol (c) im Gewichtsverhältnis (a) : (b) : (c) = 10 : ( 7 - 8) : (0,5 - 1,5) darin enthalten ist.

2. Zahnreinigungsgel nach Anspruch 1, **dadurch gekennzeichnet, daß** Wasser (d) und Feuchthaltemittel (a + b + c) im Gewichtsverhältnis (d) : (a + b + c) = 1 : (2 - 3) enthalten sind.

3. Zahnreinigungsgel nach einem der Ansprüche 1 oder 2, **dadurch gekennzeichnet, daß** zur Erzielung eines ansprechenden Aussehens gefärbte Kieselsäurepartikel in einer Menge von 0.1 - 1,0 Gew.-% darin enthalten sind.

4. Zahnreinigungsgel nach einem der Ansprüche 1 - 3, **dadurch gekennzeichnet, daß** als weitere Zahnpasteninhaltsstoffe Bindemittel, Tenside, Geschmacksstoffe und Wirkstoffe gegen Zahn- und Zahnfleischerkrankungen enthalten sind.

5. Zahnreinigungsgel nach einem der Ansprüche 1 - 4, **dadurch gekennzeichnet, daß** als Bindemittel Xanthan-Gum, Carboxymethylcellulose, Polyvinylpyrrolidon oder ein Gemisch dieser wasserlöslichen Polymeren in einerMenge bis zu 0.5 Gew.-% enthalten ist.

6. Zahnreinigungsgel nach Anspruch 4 oder 5, **dadurch gekennzeichnet, daß** als Wirkstoffe Fluorverbindungen, antimikrobielle Stoffe, Vitamine und Mineralsalze enthalten sind.

## Claims

1. A liquid transparent tooth cleaning preparation containing
10 to 15% by weight of silica polishing components,
55 to 70% by weight of humectants,
15 to 30% by weight of water and
2 to 10% by weight of other toothpaste ingredients,
**characterized in that** a mixture of sorbitol (a), glycerol (b) and polyethylene glycol (c) in a ratio by weight of (a) to (b) to (c) of 10 : (7-8) : (0.5-1.5) is present as the humectant.

2. A tooth cleaning gel as claimed in claim 1, **characterized in that** water (d) and humectant (a+b+c) are present in a ratio by weight of (d) to (a+b+c) of 1 : (2-3).

3. A tooth cleaning gel as claimed in claim 1 or 2, **characterized in that**, to obtain an attractive appearance, colored silica particles are present in a quantity of 0.1 to 1.0% by weight.

4. A tooth cleaning gel as claimed in claims 1 to 3, **characterized in that** binders, surfactants, flavors and substances active against diseases of the teeth and gums are present as further toothpaste ingredients.

5. A tooth cleaning gel as claimed in any of claims 1 to 4, **characterized in that** xanthan gum, carboxymethyl cellulose, polyvinyl pyrrolidone or a mixture of these water-soluble polymers is present in a quantity of up to 0.5% by weight.

6. A tooth cleaning gel as claimed in claim 4 or 5, **characterized in that** fluorine compounds, antimicrobial agents, vitamins and mineral salts are present as active substances.

## Revendications

1. Gel de nettoyage dentaire liquide, transparent, contenant
10-15 % en poids de composant de polissage à base d'acide silicique
55-70 % en poids d'agent de rétention d'humidité
15-30 % en poids d'eau et
2-10 % en poids d'autres constituants de dentifrice,
**caractérisé en ce que**, comme agent de rétention d'humidité, il contient un mélange de sorbitol (a), de glycérol (b) et de polyéthylèneglycol (c) dans un rapport pondéral de (a) : (b) : (c)=10 : (7-8) : (0,5-1,5).

2. Gel de nettoyage dentaire selon la revendication 1, **caractérisé en ce qu'**il contient l'eau (d) et l'agent de rétention d'humidité (a+b+c) dans un rapport pondéral de (d) : (a+b+c)= 1 : (2-3).

3. Gel de nettoyage dentaire selon la revendication 1 ou 2, **caractérisé en ce que**, pour présenter un aspect attractif, il contient des particules colorées d'acide silicique dans une proportion de 0,1-1,0 % en poids.

4. Gel de nettoyage dentaire selon l'une quelconque des revendications 1 à 3, **caractérisé en ce que**, comme autres constituants de dentifrice, il contient des liants, des tensioactifs, des agents de goût et des principes actifs contre les maladies des dents et de la gencive.

5. Gel de nettoyage dentaire selon l'une quelconque des revendications 1 à 4, **caractérisé en ce que**, comme liant, il contient de la gomme xanthane, de la carboxyméthylcellulose, de la polyvinylpyrrolidone ou un mélange de ces polymères hydrosolubles en une quantité pouvant aller jusqu'à 0,5 % en poids.

6. Gel de nettoyage dentaire selon la revendication 4 ou 5, **caractérisé en ce que**, comme principe actif, il contient des composés du fluor, des substances antimicrobiennes, des vitamines et des sels minéraux.
